# EUROPEAN PATENT SPECIFICATION

(11) **EP 2 421 484 B1**
(45) Date of publication and mention of the grant of the patent: **06.11.2019**
(21) Application number: 10728283.2
(22) Date of filing: 21.04.2010
(51) Int. Cl.: A61F 2/88, A61F 2/91, A61F 2/07

(54) **HELICAL HYBRID STENT**
SPIRALFÖRMIGER HYBRIDSTENT
STENT HYBRIDE HÉLICOÏDAL

(30) Priority: 22.04.2009 US 428347
(43) Date of publication of application: 29.02.2012
(60) Divisional application: 12176459.1
(73) Proprietor: Medinol Ltd., Tel Aviv 6158101 (IL)
(72) Inventor: RICHTER, Jacob, 46920 Arsuf (IL)
(74) Representative: Kuhnen & Wacker Patent- und Rechtsanwaltsbüro PartG mbB
(86) International application number: PCT/IB2010/001036
(87) International publication number: WO 2010/122424

(56) References cited:
- WO-A1-98/41169
- WO-A1-03/057077
- WO-A2-2008/049045
- US-A1- 2006 178 727
- US-B1- 6 503 270

## Description

### FIELD OF THE INVENTION

The invention relates to a stent having a tubular structure comprising: a coiled main stent component having first and second side bands, wherein each of the side bands has an undulating pattern, wherein the first and second side bands are intermittently connected; and first and second end bands having an undulating pattern, wherein said first and second end bands form right cylinders to a longitudinal axis of the main stent component at the longitudinal ends of the stent.

Such a stent is known from US 2006/0178727 A1.

### BACKGROUND OF THE INVENTION

Various stents are known in the art. Typically, stents are generally tubular in shape, and are expandable from a relatively small, unexpanded diameter to a larger, expanded diameter. For implantation, the stent is typically mounted on the end of a catheter with the stent being held on the catheter in its relatively small, unexpanded diameter. Using a catheter, the unexpanded stent is directed through the lumen to the intended implantation site. Once the stent is at the intended implantation site, it is expanded, typically either by a balloon or by allowing the stent to self-expand. In either case, the expanded stent resists the tendency of the vessel to narrow, thereby maintaining the vessel's patency.

Stents may be constructed from tubes or from a flat sheet of metal, which is rolled and fixed such as by welding, mechanical lock or otherwise, to form the tubular structure of the stent.

Some examples of patents relating to stent designs include U.S. Patent No. 4,733,665 to Palmaz; U.S. Patent No. 4,800,882 and 5,282,824 to Gianturco; U.S. Patent Nos. 4,856,516 and 5,116,365 to Hillstead; U.S. Patent Nos. 4,886,062 and 4,969,458 to Wiktor; U.S. Patent No. 5,019,090 to Pinchuk; U.S. Patent No. 5,102,417 to Palmaz and Schatz; U.S. Patent No. 5,104,404 to Wolff; U.S. Patent No. 5,161,547 to Tower; U.S. Patent No. 5,383,892 to Cardon et al.; U.S. Patent No. 5,449,373 to Pinchasik et al.; and U.S. Patent No. 5,733,303 to Israel et al.

One type of stent is known as the helical or coiled stent. Such stent designs are described in, for example, U.S. Patent Nos. 6,503,270 and 6,355,059. This stent design is configured as a helical stent in which the coil is formed from a wound strip of cells wherein the cells form a serpentine pattern comprising a series of bends. Other similar helically coiled stent structures are known in the art.

Prior stent designs have focused on providing sufficient radial strength when it is expanded so that it can sufficiently support the lumen. Stents with high radial strength, however, tend also to be more longitudinally stiff than the vessel in which it is implanted. When the stent is more longitudinally stiff than the vessel in which it is implanted, increased trauma to the vessel may occur at the ends of the stent, due to stress concentrations on account of the mismatch in compliance between the stented and un-stented sections of the vessel, or otherwise. In addition, the stent may interfere with the vessel's natural tendency to bend and to stretch. Conversely, stents with good flexibility often lack sufficient and/or uniform radial support for the vessel wall. Thus, a continued need exists in the art for a stent having a balance of good radial strength and a high degree of longitudinal flexibility.

Another problem in the art arises when trying to simplify the manufacturing process of a stent to reduce costs yet prevent manufacturing defects, while still producing a stent with uniformly high flexibility and sufficient radial support.

US 6 503 270 B1 describes a stent formed from a strip comprising first and second side bands, maintained in a tubular structure by welding. The strip is tapered at its ends, such that the undulating pattern of the side converge at ends This results in tip ends on either side of the strip. Thus, the problem to be solved is providing a flexible stent with smooth ends.

US 2006/0178727 A1 describes a coiled ribbon stent and a coiled ladder stent formed from a strip which is helically wound to form a tubular structure. The tubular structure is wrapped or coated with a biocompatible material in order to maintain the stent in its tubular configuration.

### SUMMARY OF THE INVENTION

To overcome the aforementioned drawback the stent of the present invention is characterized by the features of claim 1.

Further improvements are subject to the dependent claims.

Upon formation into a stent, the main stent component forms a tubular structure of helical windings at an oblique angle to the longitudinal axis of the stent. The helical windings extend at least along the central portion of the stent. Each winding may be spaced apart from the adjacent winding, or may be nestled in the adjacent winding. The distance along the longitudinal axis of the stent between the windings may be varied depending on the needs of the particular stent. Before it is helically wound to form a tubular stent, the main stent component is a flat ribbon or strip, which is defined by undulations (*e.g.*, sinusoidal, zig-zag) or a patterned band of cells (*e.g.*, hexagonal or other like-geometric structure). The strip is composed of one or more side bands that wind helically along the length of the stent, as well as end bands extending from either or both ends of the side bands, positioned at an angle to the side bands. The end bands are configured to form a right cylinder to the longitudinal axis of the stent at either or both ends of the formed stent. The side bands and end bands are understood to include portions having, for example, a generally sinusoidal, zig-zag, hexagonal or other like geometric structure.

In one embodiment of the invention, the main stent component may have one or more struts, which are sufficiently wide to include one or more full or partial fenestrations. The fenestrated struts may be connected by loops or turns whose width is narrower than that of the fenestrated struts to provide enhanced flexibility in the loops or turns.

In another embodiment, the main stent component may be designed such that each helical winding is nestled next to an adjacent helical winding of the helical structure so that the space between the windings is minimized; that is, one winding is nestled into an adjacent, substantially similar winding as the side band ribbon travels diagonally around the circumference of the stent. In this manner, the helical windings of the stent provide enhanced coverage of the wall of the lumen without loss of overall stent flexibility. Because the helical windings may be nestled into one another without directly touching each other, the overall flexibility of the formed stent is unaffected by the proximity of adjacent windings of the helical coils.

The second stent component, *i.e.*, a securement, functions to maintain the tubular shape of the main stent component while aiding in longitudinal flexibility. The securement provides structural support to the stent. The securement is oriented and affixed to the main stent component such that, upon expansion or bending of the stent, the securement contributes to the overall flexibility of the stent while still maintaining the main stent component in a tubular shape. The securement may comprise fibers, wires, threads, ribbons, strips, polymers, meshes or the like.

### BRIEF DESCRIPTION OF THE DRAWINGS

Figure 1 illustrates a main stent component composed of a flat ribbon with a patterned band according to one embodiment of the invention.
Figure 2 illustrates a main stent component of a patterned band formed into a tubular shape having a space between adjacent helical windings.
Figure 3 illustrate another embodiment wherein a helical winding of the main stent component is nestled into an adjacent helical winding.
Figure 4 illustrates an embodiment of a main stent component composed of a flat ribbon having a patterned band and comprises struts with one or more exemplary fenestrations.
Figure 4A is an enlarged view of an end band of the main stent component of Figure 4.
Figure 5 illustrates a main stent component composed of a flat ribbon having undulations and comprising struts with one or more exemplary fenestrations.
Figure 5A is an enlarged view of a first end band of the flat ribbon of Figure 5.
Figure 5B is an enlarged view of a second end band of the flat ribbon of Figure 5.
Figure 6 illustrates a photograph of a securement structure and a main stent component.
Figure 7 illustrates an embodiment of a helical main stent component with a patterned band and embedded in a securement.
Figure 8 illustrates an embodiment of the helical main stent component embedded in several ribbon securements.
Figure 9 illustrates a helical main stent component maintained by a plurality of helical securements fastened at discrete points.

### DETAILED DESCRIPTION OF THE INVENTION

This invention provides a new class of intraluminal prosthetic devices defined as helical hybrid stents. As further explained below, the stents of the invention comprise a main stent component in the form of a helical tubular structure, which may be held in the tubular shape by a second stent component, *i.e.*, a securement. The main stent component is formed from a continuous undulating or patterned strip helically wound to form a helical stent. The strip has end sections that form cylindrical rings in the tubular helical stent. As such, one inventive feature is the central body of the stent having a spiral structure which is flanked by cylindrical rings at both ends of the stent. The strip forming the central body portion comprises one or more side bands each having undulations while the ends sections comprise one or more end bands each having undulations. Each end band is connected to a side band at an angle offset to the side band and may extend back toward the side band. The lengths of struts forming the undulations of the side or end bands may be uniform or variable to assist in the helical winding. The side and end bands may be connected to one another by cross-struts. The length and shape of the cross-struts may be varied across the length of the strip, and the cross-strut may contain one or more loops. The undulations of the helically wound stent may be nestled next to an adjacent helical undulation to promote uniform coverage. The main stent component may be formed of any conventional metallic substance or an amorphous metal alloy.

In any of the novel helical stents herein, the main stent component may also include struts having full or partial fenestrations of any suitable shape and size in the side and/or end bands. Fenestrations allow the deposit of a therapeutic agent with clinical benefits within the recesses of the helical stent. The stent may be configured with struts of sufficient width to accommodate the fenestrations and loops connecting the struts of sufficiently narrow dimensions to accommodate bending and expansion of the resulting stent.

The main stent component, with or without fenestrations or nestling, is held in place by another inventive feature, *i.e*., the securement. The securement may comprise any form of polymer, fibers, wires, threads, bands, ribbons, meshes or sheets affixed to the main stent component by any of a variety of means known in the art, such as, for example, welding, bonding, embedding, braiding, weaving, crimping, tying, press-fitting or injection into a mold with the main stent component, also including joining by adhesive means, *e.g*., gluing, dip coating, spray coating, or the like. The securement may be oriented circumferentially, helically, longitudinally or otherwise and may be affixed to or embedded in a portion or the entirety of the main stent component such that, upon expansion or bending of the stent, the securement facilitates the overall flexibility of the stent while still maintaining the main stent component in a tubular shape.

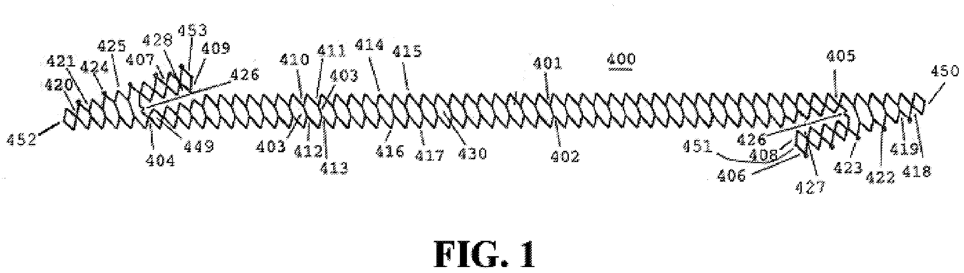

The main stent component may be formed and patterned from a strip using methods known in the art or described herein. FIG. 1 illustrates an embodiment of the invention wherein the main stent component is formed as a patterned band, shown in an uncoiled state, *i.e*., flat. As depicted in FIG. 1, the pattern of the ribbon comprises a single row of cells 430, defined by an interconnected first side band 401 with undulations and a second side band 402 with undulations. Also, end bands 406 and 407 extend at an oblique angle to side bands 401 and 402. As the main stent component is formed into a tubular shape, the row of cells 430 take on a continuous helical winding along the central portion of the longitudinal axis of the stent while end bands 406 and 407 form a right cylindrical shape at each end of the stent. The formation of the tubular helical central body portion from a flat patterned band in this manner is distinguished from the process where the design is formed from a pre-existing tubular material.

In FIG. 1, the first side band 401 and second side band 402 extend in a generally parallel orientation except at either end of the side bands where the first side band tapers toward the second side band and the second side band tapers toward the first side band. As depicted in FIG. 1, the undulating pattern of the first side band 401 comprises troughs (*e.g.*, 410, 411) that extend toward the second side band 402 and peaks (*e.g.*, 414, 415) that point away from the second side band 402. Similarly, the undulations of the second side band 402 comprise troughs (*e.g.*, 412, 413) that extend toward the first side band 401 and peaks (*e.g.*, 416, 417) that point away from the first side band 401. Thus, in FIG. 1, the first and second side bands are oriented out-of-phase with each other (troughs substantially aligned with troughs). In other embodiments, the first and second side bands may be in-phase so that the peaks and troughs are substantially aligned.

In FIG. 1, the struts of the side bands defining peaks (416, 417) and troughs (412, 413) have substantially equal lengths in the central strip portion but become shorter as the side bands taper toward each other. In the embodiment of FIG. 1, the first side band 401 and second side band 402 are connected to each other by a plurality of first cross-struts 403, defining a row of cells. Specifically, at least one trough (*e.g.*, 411) of the first side band 401 is connected to a corresponding trough (*e.g.*, 413) of the second side band 402 via a first cross strut 403 in FIG. 1. Thus, in this embodiment, a series of cells are formed, each cell 430 defined individually by the joining of the adjacent side bands to form an enclosed space by cross-struts. For example, a cell in the central strip portion is defined by the portion of the first side band between troughs 410 and 411, the portion of the second side band between troughs 412 and 413 and first cross-struts 403 respectively connecting troughs 410 and 412 and troughs 411 and 413.

In alternative embodiments, the number, length and shape of first cross-struts 403 may differ from that illustrated in FIG. 1. For example, the first cross-struts 403 may connect the first band 401 and second band 402 at regular intervals at, *inter alia*, every trough, every second trough, every third trough, or every fourth trough, etc., thereby making larger cells or cells with different geometric properties. Also, cross-struts may be omitted and the first side band may be connected directed to the second side band. For example, cell 449 is diamond shaped and defined only by the undulations of the first and second side bands. In other embodiments, the first cross-struts 403 may connect the first side band 401 and second side band 402 at varying, non-regular intervals. Variable, non-regular interval connections may form a variety of differently sized cells along a continuous main stent component as may be appropriate for a particular use. Further, the cross-struts may connect the peaks of the first side band to the trough of the second side band, or the troughs of the first side band to the peaks of the second side band.

The first cross-struts 403 may each have the same width relative to each other and to the side bands 401, 402, as shown in FIG. 1. Alternatively, the first cross-struts 403 may have a different width from the first and second side bands 401, 402, or a different width from each other, as appropriate for a particular use. The cross-struts may connect adjacent or offset troughs of the first and second side bands 401, 402. In addition, first cross-struts 403 may comprise a straight member or may contain one or more loops. As shown in FIG. 1, differently shaped cross-struts, or no cross-struts may alternatively be employed in a single stent design depending on the particular use of the stent so that a stent having different cell shapes may be formed. Exemplary cell shapes are described in U.S. Patent No. 7,141,062 (triangular cells) or 5,733,303 (square cells); the disclosure of square and triangular cell structures. Square cells have four points of connection as between radially supporting elements while a triangular cell has three points of connection between radially supporting elements. The undulations of the side and end bands provide the radially supporting elements of the invention.

The side bands 401 and 402 of the main stent component 400 in the embodiment depicted in FIG. 1 taper at each end. The length of the cross-struts 403 shorten toward each end of the main stent component 400, so that the first and second side bands 401, 402 become more snugly arranged and eventually are connected directly at points of connection 404 and 405. Alternatively, or in addition to shortened cross-struts, the side bands may taper to one another by reducing the strut lengths in the undulations.

Extending from the end of either or both side bands 401 and 402 in FIG 1 are end bands 406 and 407. Thus, a first end band 406 extends from the end of the first side band 401 in a direction offset and oblique from the general direction of the first side band 401. In FIG. 1, the first end band extends back in the direction of the first side band at an angle less than 45 degrees to the direction of the first side band when the strip is laid flat. A second end band 407 extends from the end of the second side band 402 in a general direction offset and oblique from the general direction of the second side band 402 and opposite the first end band. In FIG. 1, the second end band also extends back in the direction of the second side band at an angle less than 45 degrees to the direction of the second side band when the strip is laid flat. When forming the tubular stent, end bands 406 and 407 are configured to form right cylinders at the ends of the stent, and flank both ends of the helical winding of the strip. First end band 406 has first edge 450 and second edge 451. In the tubular form, first edge 450 is brought together with second edge 451 to form a right cylinder to the longitudinal axis of the stent. Second end band 403 has first edge 452 and second edge 453. In the tubular form, first edge 452 is brought together with second edge 453 for form a right cylinder to the longitudinal axis of the stent. As further explained below, edge 450 and 451 may be permanently connected, or as an alternative, may be held in position with a securement, which may hold the two edges in close proximity to form a right cylinder to the longitudinal axis of the stent.

The first end band 406 and second end band 407 each contain undulations having struts and loops or turns. The first end band 406 has troughs (*e.g*., 418, 419) that extend toward the first side band 401 and peaks (*e.g.*, 422, 423) that point away from the first side band 401. Likewise, the second end band 407 has troughs (*e.g.*, 420, 421) that extend toward the second side band and peaks (*e.g.*, 424, 425) that point away from the second side band 402. The first end band 406 connects directly to the first side band 401 at, *e.g.*, trough 418; however, as the first end band 406 angularly extends away from the first side band, second cross-struts 426 connect the first end band 406 to the first side band 401. Likewise, the second end band 407 connects directly to the second side band 402 at, *e.g.*, trough 420; however, as the second end band 407 angularly extends away from the second side band, second cross-struts 426 connect the second end band 407 to the second side band 402. As depicted in FIG. 1, the second cross-struts 426 may contain one or more loops between points of connection with adjacent end bands and/or side bands. The peaks of the first end band 406 and second end band 407 optionally may have additional circular structures extending from the peaks (*e.g.*, 423, 424) as depicted by FIG. 1.

In addition, a third end band 408 having undulations is arranged generally parallel to first end band 406, with the troughs of the third end band, *e.g.*, 427, extending toward the first end band and directly connected to said first end band. A fourth end band 409 having undulations is arranged generally parallel to second end band 407, with the troughs of the fourth end band, *e.g.* 428, extending toward the second end band.

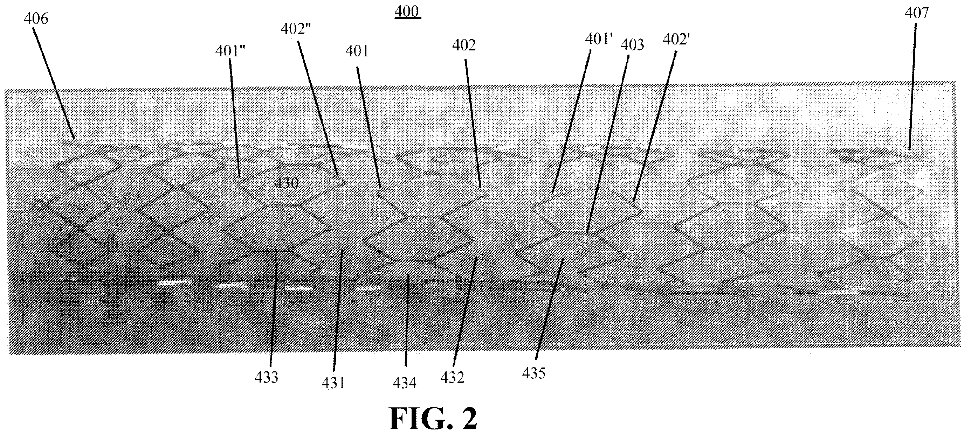

FIG. 2 illustrates the main stent component 400 of FIG. 1 helically wound into a tubular structure. The strip of cells 430 in main stent component 400 forms a helical winding in the central portion in which a cell in one winding is longitudinally-spaced apart from the cell of an adjacent winding. First side band 401 and second side band 402 are connected by cross-struts 403 and are helically wound around the longitudinal axis of the stent so that first and second side bands alternate along the stent's longitudinal axis. In the helically wound stent, as shown in FIG. 2, first side band 401 is adjacent and connected to adjacent second side band 402 by cross strut 403, but is also adjacent and unconnected to the second side band of an adjacent winding 402". Similarly, second side band 402 is unconnected to a longitudinally adjacent first side band 401' in the helical stent. End bands 406 and 407 secure the ends of the tubular structure and form substantial right cylindrical rings relative to the longitudinal axis of the stent. Open spaces, *e.g.*, 431 and 432, exists between adjacent windings, *e.g*., 433, 434, 435. In FIG. 2, the first and second side bands 401 and 402 are spaced apart either by the presence of the cross-struts, or by the spaced winding of the helical strip. In addition, a securement, as discussed below, may maintain the spacing between the adjacent windings.

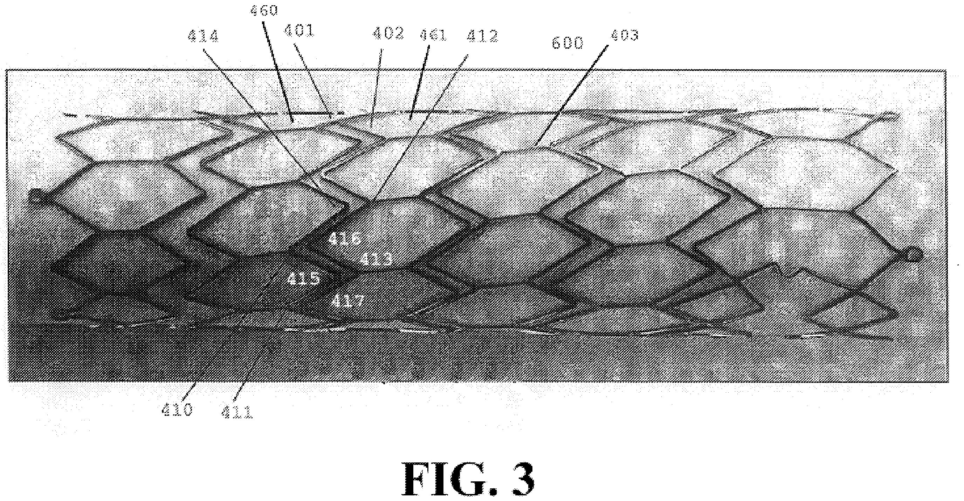

FIG. 3 illustrates a stent according to another embodiment of the invention wherein the helical windings are positioned so that little or no substantial longitudinal space exists between cycles of the helical coils. That is, as illustrated by FIG. 3, the peaks (*e.g.* 414, 415) of the first helical winding 460 are nestled toward troughs (*e.g.* 412, 413) of the second helical winding 461; yet, the first side band 401 remains substantially parallel to the second side band 402. Likewise, the peaks (*e.g.* 416, 417) of the second helical winding 461 are nestled toward troughs (*e.g.* 410, 411) of the first helical winding 460. It may be desirable to position the nestled side bands so that no direct contact occurs between first side band 401 and second side band 402. Because the first side band 401 and the second side band 402 are arranged so that the substantially similar but opposite undulations are aligned, the first side band 401 and the second side band 402 can approach one another in this fashion over the entire length of the formed stent. Side bands having struts of identical lengths also aid in nestling. In this manner, the first side band 401 and the second side band 402 may be described as nestled to one another. In FIG. 3, the first and second side bands 401 and 402 are spaced apart only by the presence of the cross-struts. As shown, the distance between the nestled windings is shorter than the length of cross-strut 403. Further, the first side band is nestled toward helically adjacent second side band without direct connection between the first side band and adjacent second side band. The stent of FIG. 3 has the additional advantage that the nestling of adjacent windings minimizes the unsupported areas of the vessel wall and/or securement to prevent sagging of the securement into the lumen upon expansion without any loss of flexibility to the stent, as further discussed below. In addition, the nestling of the helical coils separately may facilitate the maintenance of the structure in the tubular form.

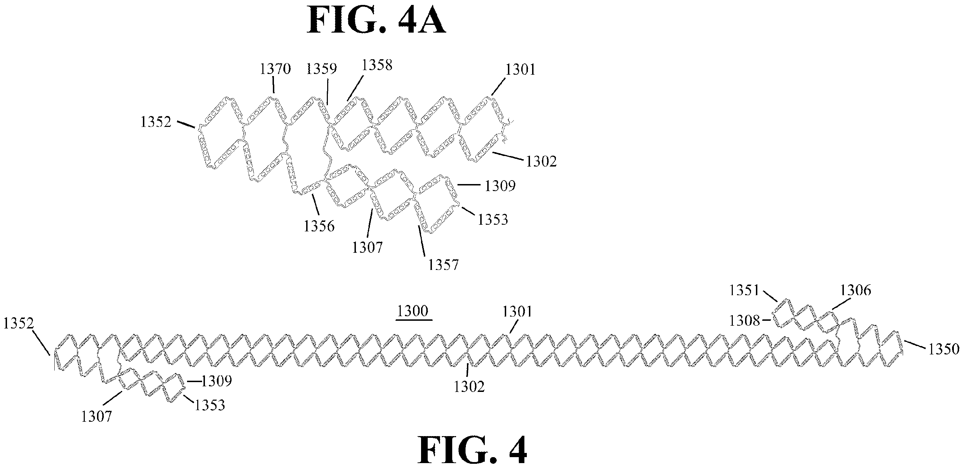

FIG. 4 illustrates an alternative embodiment wherein the main stent component 1300 is laid out in flat form, *i.e.*, uncoiled. As depicted, the main stent component 1300 has a patterned band in the longitudinal direction. Like the embodiment of FIG. 1, the design of the main stent component 1300 in FIG. 4 contains a first side band 1301, a second side band 1302, a first end band 1306, a second end band 1307, a third end band 1308 and a fourth end band 1309. In the tubular form, side bands 1301 and 1302 form a continuous helical winding for the central portion of the stent body while first and second end bands 1306 and 1307 form right cylinders to the longitudinal axis of the stent for the end rings of the stent. In the first end band, first edge 1350 is brought together with second edge 1351 while, in the second end band, first edge 1352 is brought together with second edge 1353. Main stent component 1300 comprises struts having one or more fenestrations into which a therapeutic substance may be deposited.

Each band is formed with struts of sufficient width to include one or more fenestrations as shown, for example, in FIG. 4. The fenestrated struts of main stent component 1300 may be of any geometric shape, including, but not limited to, round, oval or rectangular. Further, the fenestrations may extend through the entire thickness of the strut (full fenestrations), or may extend only partially through (partial fenestrations), being open only on one side of the strut (luminal or abluminal in the tubular form). Also, the stent may have struts containing fenestrations having variable sizes, numbers and shapes on one strut or between different struts. The invention contemplates struts having full and/or partial fenestrations on either or both of the side and/or end bands. The struts defining the peaks and troughs of the side bands may vary in length along the length of the main stent component to accommodate the desired shape for the resulting helically coiled stent structure and the number of fenestrations. For example, in FIG. 4A, side band struts 1358 and 1359 differ in length as do end band struts 1356 and 1357. The fenestrated struts are connected by loops or turns 1370 wherein the material is narrower than that of the fenestrated struts to provide enhanced flexibility.

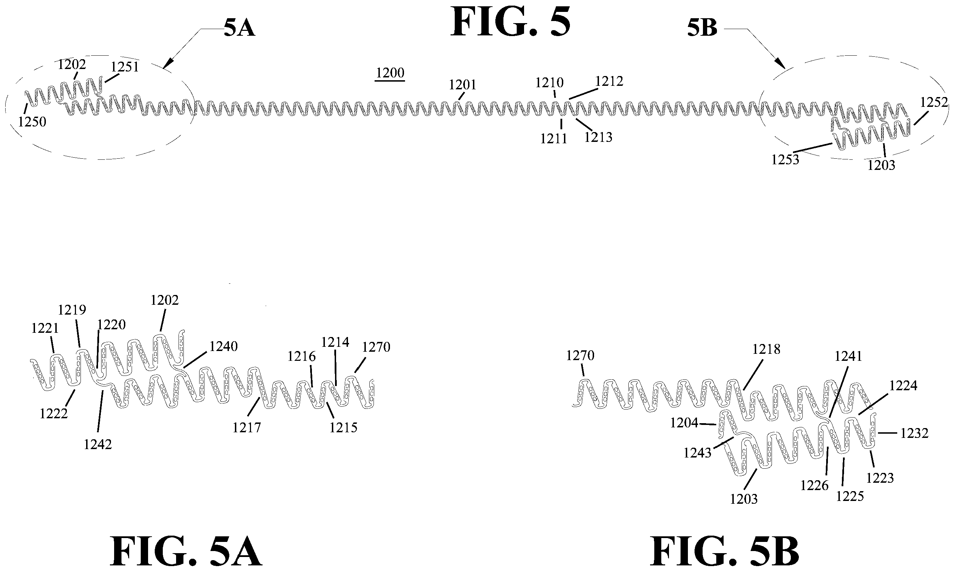

FIG. 5 illustrates yet another embodiment of the invention where the main stent component 1200 is laid out in flat form, *i.e.*, uncoiled. As depicted, the main stent component 1200 is a single side band 1201 in the longitudinal direction when laid flat. Side band 1201 is attached to first end band 1202 and second end band 1203 by cross-struts 1240 and 1241, respectively. Side band 1201 comprises an alternating pattern of peaks (*e.g.*, 1210, 1212) and troughs (*e.g.*, 1211, 1213) defined by struts having the same or variable lengths. Each side and end band is formed with struts having sufficient width to include one or more full or partial fenestrations, as described above for FIG. 4, and are also applicable to FIG. 5. The fenestrated struts are connected by loops or turns 1270 that are narrower than that of the fenestrated struts to provide enhanced flexibility. As shown in FIG. 5A, the struts are of varying length and vary in the number of fenestrations in each strut. For example, strut 1217 has a different length and number of fenestrations than strut 1215. Strut 1216 has a different length but the same number of fenestrations than strut 1215. And struts 1214 and 1215 have the same lengths and number of fenestrations. The stent of FIG. 5A contemplates that struts (*e.g*., 1217) near the ends of the first side band 1201 may have different lengths than struts 1214 and 1215 and are configured to aid in helical winding.

End bands 1202 and 1203 form circumferential end rings upon rolling of the structure into a stent. The first end band 1202 and second end band 1203 extend from the ends of the side band 1201 in a direction angularly offset from the general direction of the side band 1201. End bands 1202 and 1203 are configured to form right cylinders at the ends of the stent, flanking the helical windings of the central stent body upon winding of the structure into a stent. First end band 1202 has first edge 1250 and second edge 1251. In the tubular form, first edge 1250 is brought together with second edge 1251 to form a right cylinder to the longitudinal axis of the stent. Second end band 1203 has first edge 1252 and second edge 1253. In the tubular form, first edge 1252 is brought together with second edge 1253 to form a right cylinder to the longitudinal axis of the stent. As further explained below, the edges (1250 and 1251; 1252 and 1253) may be permanently affixed, or as an alternative, may be held in position with a securement which may keep the two edges in close proximity to maintain a right cylinder to the longitudinal axis of the stent.

In FIG. 5A, first end band 1202 comprises a band of undulations. The direction of the first end band 1202 is offset at an angle to the direction of the side band 1201. In FIG. 5A, the first end band extends from the side band is at an angle less than 45 degrees to the central body of the stent when the stent is laid flat. The undulating pattern of the first end band 1202 comprises alternating peaks (*e.g.*, 1219, 1221) and troughs (*e.g.*, 1220, 1222). Troughs (1220, 1222) of the first end band extend in the direction of the side band while the peaks (1219, 1221) point away from the side band. First end band 1202 also may contain struts having fenestrations. In FIG. 5A, cross-links 1240 and 1242, for example, connect the side band to the first end band. Cross-links 1240 and 1242 extend from the troughs of the first end band to the peak of the side band. Cross-links extending between the side band and the first end band are flexible connectors having one or more curved portions. The invention also contemplates an embodiment where the cross-links may contain one or more loops.

In FIG. 5B, second end band 1203 also comprises a band of undulations. The direction of the second end band 1203 is angularly offset to the direction of the side band 1201. Preferably, the second end band extends from the side band at an angle less than 45 degrees to the central body of the stent when the stent is laid flat. The undulating pattern of the second end band 1203 comprises alternating peaks (*e.g.*, 1223, 1225) and troughs (*e.g.*, 1224, 1226). Troughs (1224, 1226) of the second end band extend in the direction of the side band while the peaks (1223, 1225) point away from the side band. Second end band 1203 contains struts having fenestrations. In FIG. 5B, cross-link 1241 connects the side band to the second end band. Cross-link 1241 extends from the trough of the second end band to the trough of the side band. Cross-links extending between the side band and second end band are flexible connectors having one or more curved portions. Cross-links connecting the side band to the second end band may comprise at least one loop.

In addition, the invention contemplates other end bands similar in construction to first and second end bands and connected to either the first or second end bands to facilitate helical winding and uniform coverage. In FIG. 5B, a third end band 1204 having fenestrated struts is connected to the second end band by cross-link 1243. As illustrated in FIGS 5A and 5B, the invention contemplates first and second end bands which are not identically connected to the undulating or patterned side bands and which are not identical to each other. Like the side band, any one or all the end bands may comprise struts sufficiently wide to accommodate one or more full or partial fenestrations which are connected together with loops having a narrower gauge than the fenestrated struts.

The main stent component is held in a helically wound position by a second component, securing the helical windings into a tubular structure. The second component, referred to herein as a securement, may be one or more of a variety of means for securing the main stent component in the tubular form. The securement maintains the helical winding of the central stent body and/or the formation of right cylinders by the end bands. The securement comprises a structure in the form of fibers, sheets, threads or ribbons which are wrapped around or itself embedded in the coiled main stent component. In another embodiment, wires or ribbons formed of a metal or non-metal material maintain the main stent component in its tubular configuration. The securement comprises a material that allows flexibility and expansion of the helical main stent component without tearing or detachment of the securement and allows movement between the coiled windings of the main stent body relative to each other. Such a material may be applied to a tubular stent in a continuous or non-continuous manner depending upon the particular needs of the structure contemplated.

Preferably, the securement allows expansion of the stent and maximal bending during and after implantation without reaching the elastic limit. The elastic range may be a product either of inherent elasticity in the material used, such as with certain polymers, or of the inclusion of a reserve length of a non-elastic material between points of connection with the main stent component. Yet another advantage of a securement is the prevention of "stent jail" phenomenon, or the complication of tracking into side branches covered by the stent. A further advantage is the high fatigue resistance of particular securement structures with high elastic range.

In one embodiment, the securement is a polymer that is a biocompatible material. Biocompatible material may be durable, such as polyesters, polyanhydrides, polyethylenes, polyorthoesters, polyphosphazenes, polyurethane, polycarbonate urethane, silicones, polyolefins, polyamides, polycaprolactams, polyimides, polyvinyl alcohols, acrylic polymers and copolymers, polyethers, celluiosics and any of their combinations in blends or as copolymers. Of particular use may be silicone backbone-modified polycarbonate urethane and/or expanded polytetrafluoroethylene (ePTFE). Any polymer having a high elastic ratio (high elongation factor within the elastic range) is particularly suitable for a securement. The polymer may also be porous. In embodiments where the polymer a continuous structure with small inter-fiber distance, it may also be used as a matrix for eluting drug thereby providing a uniform elution bed. This type of porous securement may be applied to any other stent structure.

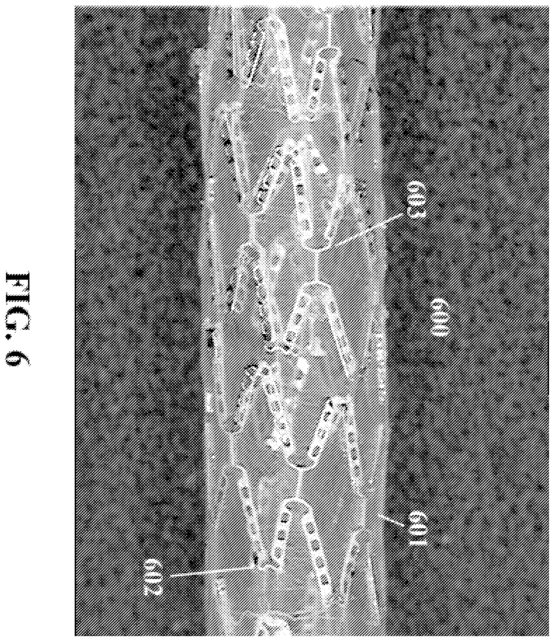

FIG. 6 shows the coiled main stent component 600 of FIG. 4, described above, wherein a porous and durable polymer securement 601 is applied over main stent component 600. Two adjacent struts of a first side band are connected to one another by turn 602, which includes a "dimple". The inclusion of a dimple in the turns is an optional feature depending upon the desired properties of the resulting stent. FIG. 6 also illustrates turn 603 which is without a dimple, and is employed in this embodiment at points where cross-struts connect the first side band to the second side band.

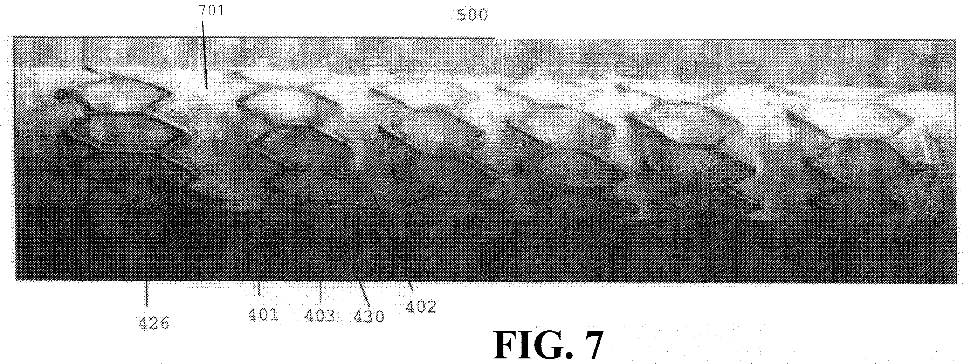

In FIG. 7, the helical main stent component 500 is secured by embedding the tubular structure in a polymer sheet 701 and illustrates one coiled form of the main stent component depicted in FIG. 2, that is, one where the adjacent helical coils of the stent are spaced apart but the stent maintains its helical structure through the embedded polymer securement. The securement may be disposed within interstices and/or embedded throughout the stent. In one embodiment, a polymer sheet may secure portions of the stent structure or may fully envelop the entire stent to hold the central portion in the helical form and the end bands in the cylindrical form.

Polymeric securements as described above may also be employed in the form of threads, wires or ribbons, thereby securing the main stent component through, for example, a series of points of connection with the main stent component. One or more securement threads, wires or ribbons may be coiled around the stent in a helically different direction than the main stent component. In particular, the thread, wire or ribbon may be coiled around the stent in the reverse helical orientation from the direction of the helically wound strip. Alternatively, securements may be arranged along a longitudinal axis of the stent. Arranged in any non-parallel direction with the main stent component, each thread, wire or ribbon may overlap with the main stent component in a regular pattern across the length of the stent and may effectively function to secure the helical stent body structure. The securement thread, wire or ribbon may be affixed to the main stent component at one or more points of overlap through a variety of means, *e.g*., welding, bonding, embedding, braiding, weaving, crimping, tying, press-fitting or the like, including also joining by adhesive means, *e.g*., gluing, dip coating, spray coating or the like. The polymeric securement may also be injected into a mold with or without the stent and hence become integrated within the stent. The threads, wires or ribbons maintain the tubular shape of the stent, while the longitudinally flexible quality of the polymeric material discussed above will enhance the overall flexibility of the stent.

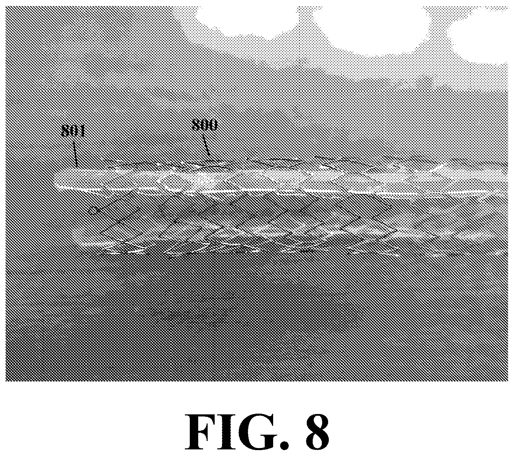

FIG. 8 illustrates a helically coiled stent wherein the main stent component 800 forms a helically wound tubular structure that is secured in place by two ribbons 801. The ribbons 801 are a polymeric material that extend along the length of the stent. The ribbons may be affixed to the outside or the inside surface of the stent, or may be embedded in the helically coiled main stent component. In FIG. 8, the main stent component 800 is embedded within each ribbon 801 at points where the main stent component 800 and each second component ribbon 801 intersect.

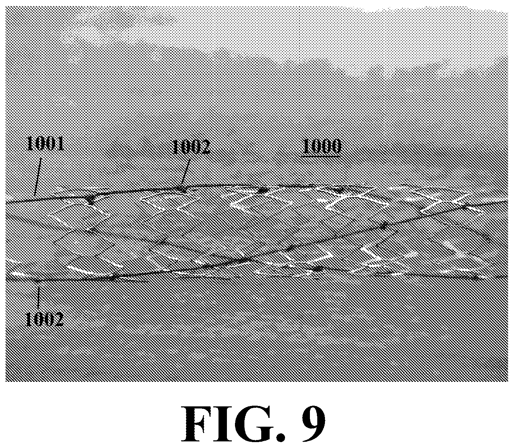

FIGS. 9 illustrate a helically coiled stent wherein the main stent component 1000 forms a tubular structure similar to FIG. 2 and one or more securement wires 1001 are coiled in a different helical direction to that of the coiled central body portion of the stent. The securement wires 1001 are affixed to the main stent component 1000 at various points of connection 1002 along the stent, thereby maintaining the helical, tubular structure.

In addition to polymeric securements, any other suitable material, including metals and/or non-metals, may be employed as securements in the form of threads, wires or ribbons to secure the main stent component. The metal or non-metal securement wire, thread or ribbon may be affixed to the main stent component where they overlap through one or more of a variety of means as identified above. If the material employed to manufacture the second component is of a lesser longitudinal flexibility than desired, increased flexibility may be achieved by increasing the length of the thread, wire or ribbon between points of connection, thereby providing reserve length of the second component that can extend upon expansion or bending of the stent.

In embodiments where the main stent component is an amorphous metal alloy, further advantages may be provided, *i.e.*, enhanced corrosion resistance, resistance to unwanted permanent deformation and/or higher strength for a given metal thickness. Stents of the invention comprising amorphous metal alloys may also exhibit significantly lower conductance or are non-conductive, compared to their crystalline or polycrystalline counterparts. Such alloys may provide improved tensile strength, elastic deformation properties, and reduced corrosion potential to the devices. These may be important features in medical devices to provide an extended fatigue-resistant lifespan for devices that are subjected to repeated deformations and fatigue in the body. In addition, these features allow production of smaller or thinner devices that are as strong as their bulkier conventional counterparts.

In another embodiment, the amorphous metal alloy of the main stent component may be a metalloid, non-limiting examples of which include silicon, boron, and phosphorus. Another possible amorphous metal alloy is a Fe-Cr-B-P alloy. Many other similar alloys are suitable and known to one of ordinary skill in the art. One embodiment of this invention contemplates intraluminal prosthetic devices comprising at least one amorphous metal alloy combined with components made of other materials, limited only by the biocompatibility of the materials. This embodiment of the invention may contain one or more amorphous metal alloys. For example, the device may have components constructed of stainless steel, cobalt chromium ("CoCr"), NiTi or other known materials. The details of these alloys, which have certain advantages, are disclosed in U.S. Patent Nos. 5,836,964 and 5,997,703.

The methods of manufacturing the amorphous metal alloys are described in U.S. Application Ser. No. 12/428,347, filed on April 22, 2009. Amorphous metal stents of the invention may be formed of one or more flat strips of helically wound metal. Because amorphous metal alloys cannot be easily welded without the metal reverting to an undesirable crystalline form, the present invention contemplates a securement for the helically wound amorphous metal alloy main stent component, further described below.

Where the main stent component is an amorphous metal alloy, the method of combining or joining the amorphous metal alloy to the securements can be achieved using particular methods known in the art. For example, a biocompatible polymer securement covering all or part of the amorphous metal main stent component may be used to secure the helical windings in its tubular shape for positioning and expansion in the lumen as well as the end bands in a cylindrical shape. Other non-limiting examples of securement methods include physical joining (*e.g*., braiding, weaving, crimping, tying, and press-fitting) and joining by adhesive methods (*e.g*., gluing, dip coating, and spray coating). Combinations of these methods are also contemplated by this invention.

As a further advantage of the invention, any or all of the securement or main stent component may be embedded with a therapeutic agent that will inhibit or decrease cell proliferation or will reduce restenosis. The main stent component may comprise at least one fenestration where the drug is deposited. Non-limiting examples of such drugs include for example sirolimus, rapamycin, everolimus and paclitaxol, and analogs of these. In addition, the stent may be treated to have active or passive surface components such as agents that will be advantageous for a longer time after the stent is embedded in the vessel wall.

The stent of the present invention may be balloon expandable or self-expanding as is known in the art. When a balloon-expandable stent system is used to deliver the stent, the stent is mounted on the balloon and the catheter assembly is positioned at the implantation site. The balloon is then inflated, radially applying a force inside the stent and the stent is expanded to its expanded diameter. Alternatively, the stent may be self-expanding in which case a balloon is not needed to facilitate expansion and delivery of the stent.

The general concepts described herein can be utilized to form helical stents with different configurations than the particular embodiments described herein. It will be appreciated by persons skilled in the art that the present invention is not limited to what has been particularly shown and described above. Rather, the scope of the present invention is defined by the claims which follow.

## Claims

1. A stent having a tubular structure comprising:
a coiled main stent component (400, 1300) having first and second side bands (401, 1301; 402, 1302), wherein each of the side bands (401, 1301; 402, 1302) has an undulating pattern, wherein the first and second side bands (401, 1301; 402, 1302) are intermittently connected; and
a securement (601, 1001) providing structural support to the main stent component (400, 1300), the securement (601, 1001) comprising a structure selected from the group consisting of fibers, sheets, threads, wires and ribbons which are wrapped around or itself embedded in the coiled main stent component;
**characterized in** first and second end bands (406, 1306; 407, 1307) having an undulating pattern, wherein said first and second end bands (406, 1306; 407, 1307) form right cylinders to a longitudinal axis of the main stent component (400, 1300) at the longitudinal ends of the stent.

2. The stent according to claim 1,
wherein said first and second side bands (401, 1301; 402, 1302) are helically oriented along the longitudinal axis of the stent, said first end band (406,1306) is a first right cylinder relative to the longitudinal axis at a first end of the stent and said second end band (407, 1307) is a second right cylinder relative to the longitudinal axis of the stent at a second end of the stent.

3. The stent according to claim 1, wherein one or more of said side bands (401, 1301; 402, 1302) of the main stent component (400, 1300) contain struts having one or more fenestrations.

4. The stent according to claim 3, wherein said fenestration is filled with a therapeutic agent.

5. The stent according to claim 3, wherein some adjacent struts of the undulating pattern have the same lengths.

6. The stent according to claim 1, wherein said securement (601, 1301) is oriented around the stent in a helical direction.

7. The stent according to claim 1, wherein the securement (601, 1301) overlaps at a point of connection with the main stent component (400, 1300) periodically over the length of the stent.

8. The stent according to claim 1, wherein the securement (601, 1301) comprises a fiber mesh.

9. The stent according to claim 1, wherein the securement (601, 1301) comprises a porous material.

10. The stent according to claim 1, wherein the securement (601, 1301) comprises a durable material.

11. The stent according to claim 10, wherein the durable material is polyurethane.

12. The stent according to claim 1, wherein said securement (601, 1301) is embedded across the entire length of the helical main stent component (400, 1300).

13. The stent according to claim 1, wherein the securement (601, 1301) is affixed to the helical main stent component (400, 1300) at an end.

14. The stent according to claim 1, wherein the main stent component (400, 1300) comprises a metal.

15. The stent according to claim 14, wherein the main stent component (400, 1300) is cut from a flat sheet of metal.

16. The stent according to claim 1, wherein the main stent component (400, 1300) comprises an amorphous metal alloy.

17. The stent according to claim 16, wherein said amorphous metal alloy comprises an element selected from the group consisting of silicon, boron, and phosphorous.

18. The stent according to claim 16, wherein said amorphous metal alloy is an iron-based alloy containing Fe, Cr, B, and P.

19. The stent according to claim 16, wherein the amorphous metal alloy contains silicon.

20. The stent according to claim 16, wherein the amorphous metal alloy comprises a Fe-Cr-B-P alloy.

21. The stent according to claim 1, wherein the first and second side bands (401, 402) are connected by at least one cross strut (403).

22. The stent according to claim 21, wherein said at least one cross strut (403) has a loop.

23. The stent according to claim 1, wherein said first side band (401, 1301) is nestled next to a helically neighboring second side band (407, 1307).

24. A stent according to claim 1, wherein the first end band (406, 1306) comprises a first edge (450, 1350) and a second edge (451, 1351) and extends at an oblique angle to said first side band (401, 1301) and the second end band (407, 1307) comprises a first edge (452, 1352) and a second edge (453, 1353) and extends at an oblique angle to said second side band (402, 1302).

## Patentansprüche

1. Stent mit einer Röhrenstruktur, aufweisend:
eine gewickelte Stent-Hauptkomponente (400, 1300) mit ersten und zweiten Seitenbändern (401, 1301; 402; 1302), wobei jedes der Seitenbänder (401, 1301; 402, 1302) ein Wellenmuster aufweist, wobei die ersten und zweiten Seitenbänder (401, 1301; 402, 1302) intermittierend miteinander verbunden sind; und
eine Sicherung (601, 1001), die der Stent-Hauptkomponente (400, 1300) strukturelle Unterstützung bietet, wobei die Sicherung (601, 1001) eine Struktur aufweist, ausgewählt aus der Gruppe bestehend aus Fasern, Blechen, Fäden, Drähten und Ribbons, welche die gewickelte Stent-Hauptkomponente umwickeln oder ihrerseits darin eingebettet sind;
**gekennzeichnet durch** erste und zweite Endbänder (406, 1306; 407, 1307) mit Wellenmustern, wobei die ersten und zweiten Endbänder (406, 1306; 407, 1307) gerade Zylinder zu einer Längsachse der Stent-Hauptkomponente (400, 1300) an einem Längsende des Stents bilden.

2. Stent nach Anspruch 1,
wobei die ersten und zweiten Seitenbänder (401, 1301; 402, 1302) spiralförmig entlang der Längsachse des Stents ausgerichtet sind, das erste Endband (406, 1306) ein erster gerader Zylinder gegenüber der Längsachse an einem ersten Ende des Stents ist und das zweite Endband (407, 1307) ein zweiter gerader Zylinder gegenüber der Längsachse des Stents an einem zweiten Ende des Stents ist.

3. Stent nach Anspruch 1, wobei eine oder mehrere der ersten Seitenbänder (401, 1301; 402, 1302) der Stent-Hauptkomponente (400, 1300) Streben mit einer oder mehreren Fensterungen enthalten.

4. Stent nach Anspruch 3, wobei die Fensterung mit einem Therapeutikum gefüllt ist.

5. Stent nach Anspruch 3, wobei einige benachbarte Streben des Wellenmusters die gleichen Längen aufweisen.

6. Stent nach Anspruch 1, wobei die Sicherung (601, 1301) in einer spiralförmigen Richtung um den Stent herum ausgerichtet ist.

7. Stent nach Anspruch 1, wobei die Sicherung (601, 1301) die Stent-Hauptkomponente (400, 1300) an einem Verbindungspunkt periodisch über die Länge des Stents überlappt.

8. Stent nach Anspruch 1, wobei die Sicherung (601, 1301) ein Fasernetz aufweist.

9. Stent nach Anspruch 1, wobei die Sicherung (601, 1301) ein poröses Material aufweist.

10. Stent nach Anspruch 1, wobei die Sicherung (601, 1301) ein haltbares Material aufweist.

11. Stent nach Anspruch 10, wobei das haltbare Material Polyurethan ist.

12. Stent nach Anspruch 1, wobei die Sicherung (601, 1301) über die gesamte Länge der spiralförmigen Stent-Hauptkomponente (400, 1300) eingebettet ist.

13. Stent nach Anspruch 1, wobei die Sicherung (601, 1301) an einem Ende der spiralförmigen Stent-Hauptkomponente (400, 1300) befestigt ist.

14. Stent nach Anspruch 1, wobei die Stent-Hauptkomponente (400, 1300) ein Metall aufweist.

15. Stent nach Anspruch 14, wobei die Stent-Hauptkomponente (400, 1300) aus einem flachen Metallblech ausgeschnitten ist.

16. Stent nach Anspruch 1, wobei die Stent-Hauptkomponente (400, 1300) eine amorphe Metalllegierung aufweist.

17. Stent nach Anspruch 16, wobei die amorphe Metalllegierung ein Element aufweist, ausgewählt aus der Gruppe bestehend aus Silizium, Bor und Phosphor.

18. Stent nach Anspruch 16, wobei die amorphe Metalllegierung eine Legierung auf Eisenbasis ist, die Fe, Cr, B und P enthält.

19. Stent nach Anspruch 16, wobei die amorphe Metalllegierung Silizium enthält.

20. Stent nach Anspruch 16, wobei die amorphe Metalllegierung eine Fe-Cr-B-P-Legierung aufweist.

21. Stent nach Anspruch 1, wobei die ersten und zweiten Seitenbänder (401, 402) durch mindestens eine Querstrebe (403) miteinander verbunden sind.

22. Stent nach Anspruch 21, wobei die mindestens eine Querstrebe (403) eine Schlaufe aufweist.

23. Stent nach Anspruch 1, wobei das erste Seitenband (401, 1301) neben einem spiralförmig benachbarten zweiten Seitenband (407, 1307) gebettet ist.

24. Stent nach Anspruch 1, wobei das erste Endband (406, 1306) eine erste Kante (450, 1350) und eine zweite Kante (451, 1351) aufweist und sich in einem geneigten Winkel zu dem ersten Seitenband (401, 1301) erstreckt und das zweite Endband (407, 1307) eine erste Kante (452, 1352) und eine zweite Kante (453, 1353) aufweist und sich in einem geneigten Winkel zu dem zweiten Seitenband (402, 1302) erstreckt.

## Revendications

1. Stent ayant une structure tubulaire comprenant :
un composant de stent principal (400, 1300) hélicoïdal ayant des première et deuxième bandes latérales (401, 1301 ; 402, 1302), dans lequel chacune des bandes latérales (401, 1301 ; 402, 1302) a un motif ondulé, dans lequel les première et deuxième bandes latérales (401, 1301 ; 402, 1302) sont reliées par intermittence ; et
une fixation (601, 1001) fournissant un support structurel au composant de stent principal (400, 1300), la fixation (601, 1001) comprenant une structure sélectionnée dans le groupe constitué de fibres, feuilles, fils, fils métalliques et rubans qui sont enroulés autour ou eux-mêmes incorporés dans le composant de stent principal hélicoïdal ;
**caractérisé par** des première et deuxième bandes d'extrémité (406, 1306 ; 407, 1307) ayant un motif ondulé, dans lequel lesdites première et deuxième bandes d'extrémité (406, 1306 ; 407, 1307) forment des cylindres droits par rapport à un axe longitudinal du composant de stent principal (400, 1300) aux extrémités longitudinales du stent.

2. Stent selon la revendication 1,
dans lequel lesdites première et deuxième bandes latérales (401, 1301 ; 402, 1302) sont orientées hélicoïdalement le long de l'axe longitudinal du stent, ladite première bande d'extrémité (406, 1306) est un premier cylindre droit par rapport à l'axe longitudinal à une première extrémité du stent et ladite deuxième bande d'extrémité (407, 1307) est un deuxième cylindre droit par rapport à l'axe longitudinal du stent à une deuxième extrémité du stent.

3. Stent selon la revendication 1, dans lequel une ou plusieurs desdites bandes latérales (401, 1301 ; 402, 1302) du composant de stent principal (400, 1300) contiennent des entretoises ayant une ou plusieurs fenêtres.

4. Stent selon la revendication 3, dans lequel ladite fenêtre est remplie d'un agent thérapeutique.

5. Stent selon la revendication 3, dans lequel quelques entretoises adjacentes du motif ondulé ont les mêmes longueurs.

6. Stent selon la revendication 1, dans lequel ladite fixation (601, 1301) est orientée autour du stent dans une direction hélicoïdale.

7. Stent selon la revendication 1, dans lequel la fixation (601, 1301) se chevauche au niveau d'un point de liaison avec le composant de stent principal (400, 1300) périodiquement sur la longueur du stent.

8. Stent selon la revendication 1, dans lequel la fixation (601, 1301) comprend un treillis de fibres.

9. Stent selon la revendication 1, dans lequel la fixation (601, 1301) comprend un matériau poreux.

10. Stent selon la revendication 1, dans lequel la fixation (601, 1301) comprend un matériau durable.

11. Stent selon la revendication 10, dans lequel le matériau durable est du polyuréthane.

12. Stent selon la revendication 1, dans lequel ladite fixation (601, 1301) est incorporée sur toute la longueur du composant de stent principal (400, 1300) hélicoïdal.

13. Stent selon la revendication 1, dans lequel la fixation (601, 1301) est fixée au composant de stent principal (400, 1300) hélicoïdal au niveau d'une extrémité.

14. Stent selon la revendication 1, dans lequel le composant de stent principal (400, 1300) comprend un métal.

15. Stent selon la revendication 14, dans lequel le composant de stent principal (400, 1300) est coupé à partir d'une feuille plate de métal.

16. Stent selon la revendication 14, dans lequel le composant de stent principal (400, 1300) comprend un alliage métallique amorphe.

17. Stent selon la revendication 16, dans lequel ledit alliage métallique amorphe comprend un élément sélectionné dans le groupe constitué de silicium, bore et phosphore.

18. Stent selon la revendication 16, dans lequel ledit alliage métallique amorphe est un alliage à base de fer contenant Fe, Cr, B et P.

19. Stent selon la revendication 16, dans lequel l'alliage métallique amorphe contient du silicium.

20. Stent selon la revendication 16, dans lequel l'alliage métallique amorphe comprend un alliage Fe-Cr-B-P.

21. Stent selon la revendication 1, dans lequel les première et deuxième bandes latérales (401, 402) sont reliées par au moins une entretoise transversale (403).

22. Stent selon la revendication 21, dans lequel ladite au moins une entretoise transversale (403) a une boucle.

23. Stent selon la revendication 1, dans lequel ladite première bande latérale (401, 1301) est nichée à côté d'une deuxième bande latérale hélicoïdalement adjacente (407, 1307).

24. Stent selon la revendication 1, dans lequel la première bande d'extrémité (406, 1306) comprend un premier bord (450, 1350) et un deuxième bord (451, 1351) et s'étend selon un angle oblique par rapport à ladite première bande latérale (401, 1301) et la deuxième bande d'extrémité (407, 1307) comprend un premier bord (452, 1352) et un deuxième bord (453, 1353) et s'étend selon un angle oblique par rapport à ladite deuxième bande latérale (402, 1302).
